# EUROPEAN PATENT APPLICATION

(11) **EP 2 679 230 A1**
(43) Date of publication of application: **01.01.2014**
(21) Application number: 12750191.4
(22) Date of filing: 27.02.2012
(51) Int. Cl.: A61K 31/724, A61K 31/015, B82Y 5/00

(54) **PHARMACEUTICAL FORMULATION COMPRISING COMPOUNDS OF THE JASMONATE FAMILY**

(30) Priority: 25.02.2011 BR PI1100297
(71) Applicant: Nanocare Technologies, Inc., New York, NY 10022 (US)
(72) Inventor: FEHR PEREIRA LOPES, José, E., Sao Carlos - SP Cep: 13560-001 (BR)
(74) Representative: Mintz Levin Cohn Ferris Glovsky and Popeo LLP
(86) International application number: PCT/IB2012/000364
(87) International publication number: WO 2012/114196

(57) **Abstract**

The current invention pertains to pharmaceutical formulation including at least one jasmonate compound of the jasmonate family of compounds combined with a carrier, such as inclusion compounds, particularly those able to form nano or micro encapsulation systems. The invention generally refers to inclusion complexes which includes jasmonate compounds.

## Description

### FIELD OF THE INVENTION

The current invention refers to pharmaceutical formulations including at least one derivate of metabolism of any element of the jasmonates family, and it can be simple, modified, compound, natural and or synthetic, where such compounds can be combined with a carrier or carried, such as inclusion compounds, particularly those capable of forming nano or micro encapsulation system. The invention generally refers to inclusion complexes which includes compounds derived from the metabolism of elements of the jasmonates family.

The jasmonate compounds family (here also named jasmonate compounds) is characterized by a structure of the cyclopentanone type. It is a group of vegetable hormones that helps to regulate growth and plant development. Particular jasmonate compounds are jasmonic acid and its esters, such as methyl jasmonate (MeJA). This document, patent CA 2630666, is an example of jasmonate compounds.

Just as the hormones called prostaglandins, found in mammals, jasmonates are derived from a cyclopentanone, biosynthetic and also derived from fat acids. Prostaglandins are biosynthesized from lynolenic acid by eicosanoid via containing also a cyclopentanone ring.

In this context, the mention of jasmonate metabolism, where several compounds are included in the process, and each of them can present a function, unique or coadjuvant to the known effect of some elements belonging to the jasmonates family, already an object of the previous patent. Such molecules resulting from the metabolism of the jasmonates family can be modified artificially for that there is synergy in its action again may it be isolated or not. Quoting an example with replacements or inclusions of amine or amide molecules or any other substance that are able to increase the specific effects of such molecules and also the transformation of the cyclopentanone ring in a cyclopentanone ring. Such changes include inclusions, replacements, conjugations, increases, reductions or any other chemical processes with any other components.

Nano or micro carriers, as seen here, includes the agents that form inclusion compounds, such as cyclodextrins (CDs), those known as native α, β and γ-CD, however here in particular modified cyclodextrins and also compound cyclodextrins. This invention comprehends any system that can form inclusion processes, besides the stated cyclodextrins. In these terms we can define several molecules with carrier capacity, particles or aggregates with micro or nano particles in general, among which we can quote several polymers or alternative polymers, copolymers, liposomes, dendrimers, metallic nanospheres, mixed polymers, biopolymers, transporter carbon structures, transporter silica structures, transporter silicon structures, injectable nanocarriers micro or nanoparticles, being that all these can be carriers in its simple form as well as they can be combined or modified from the simple form or compound among them: electrically, magnetically, chemically, radioactively, physically, thermally or robotically modified.

Inclusion compounds of the invention is to attain the accumulation of the desired target, inserting cells with specific receptors or by pores by means of membrane permeability, thus enforcing the retention effects and acting as target therapy, such nanocarriers or microcarriers can be associated to antibodies, several binding peptides, biological elements, fungi, viruses and bacteria derivates, as well as protein derivates, obtained lipids from biological sources or nucleic acids that when combined with to nanocarriers can reinforce even more its ability for recognition and internalization of the carried molecule to the target tissue. Other carriers according to the invention for example, can also include nanocarriers added to activated stimuli in the extracellular means, such as nano and micro suspensions, nano tubes, nanobars and nanofilaments, nanowires, SLN cationic (carriers of solid lipidic nanoparticles) NP gelatin (nanoparticles), transporters, PLGA (polylactic acid, polyglycolic acid), NP copolymers, PLGA nanospheres, NP hydro gel structure transporter, CPP (cellular penetration peptide), PN structure transporter, micelles polymers known as immunomicelles, functionalized NPs, crystal nano structure transporters.

The invention regards the reinforced anticancer effect provided by inclusion complexes, i.e., compounds derived from the metabolism of elements of the jasmonate family in inclusion compounds, such as condition inhibiting drugs (oxygen deficiency) for neoplasic tissue hypoxia. Without binding to the theory, it is believed that jasmonate compounds and its metabolism's elements exert its effects in many major biochemical paths - amongst which: interference in DNA synthesis, such as the effect in transcription, translation, gene regulation and energy production, in anoxia conditions like the anaerobic glycolysis, that work better in hypoxia and those are the paths that are regulated and well known in the cancer cell in hypoxia state. In more detail, it is believed that the invention's effect is on the caspases (peptidase apoptosis and cysteine) which catalyses cleavage of the pro-apoptotic protein. When the inclusion complexes stated in this invention are involved in any circumstance or any way, as cellular regulating elements for example: with BH-3, the members of the BH-2 family exclusively exert their activity over the anti apoptotic members of the family (Bcl-2, Bcl-xL), any action by this invention allows for the invented composition which increases Bak and Bax to translocate for the external membrane of the mitochondria, the whole process that can act in the process of the known dysfunction and characteristic to the neoplasic cell mitochondrial membrane, cause release of pro apoptotic proteins like cytochrome c and Smac DIABLO, and antagonist to the inhibitor of apoptosis protein (IAP's).

In general the inclusion phenomenon can be characterized by the illustration reaction formation example as laid out below:
The called inclusion complexes of molecular hosts, form new compounds.

The resulting compounds with these formulas, according to this invention, are efficient system to provide degradation and physiologic metabolism members of the elements of the jasmonate family and its possible derivates to act on specific target cells These target cells can be typically characterized as cancer cells, although it can be used on other cells or targets, affected by inflammatory processes or viral, bacteria, fungi diseases, characterization and anesthetics.

Inclusion complexes of this invention act have shown efficient performance presenting a significant reduction of toxicity of these compounds resulting from the degradation or metabolism process of constituent elements of the jasmonate family and its derivates, promoting the chemical stabilization of its molecular structure, avoiding degradation «, for example hydrolysis and oxidation.

### FUNDAMENTS OF THE INVENTION

Some molecules resulting from degradation of metabolism of jasmonate family compounds are characterized by the cyclopentanone ring. Jasmonates are known as vegetable hormones produced in a situation of stress by vegetables. Qualified as cyclopentanones, jasmonates are also known potent in vitro antibodies and have been reported as efficient agent for reducing in vivo cell tumors, as stated in patent document US6469061, where a list of jasmonate family compounds is also quoted.

But this invention refers to the fact that the compounds resulting from the degradation or metabolism may exert, on their own, the effects up to now credited to the jasmonate family elements. I.e. in the physiologic degradation of the micro or nano carried compound, or the elements which will reach the desired effect is one or more sub-products resulting from the breakage of the jasmonate element. This way nano carrying of these substances would avoid other substances that may be composed or are to be formed also of the metabolic degradation of the jasmonate family elements, needs to be administered when its effects is no longer effective or even toxic. These carried molecules, activated to the expected effect can be directly guided to the desired target.

Therefore, protecting the active compounds resulting from jasmonate metabolism including them in the inclusion compounds was an obvious path avoiding that such a principle may suffer specific degradation before it reaches the desired target, by tangible metabolism. And also for it to be taken to the necessary target, the active part of which it is part of, for that dysfunction, found within the structure of the jasmonate family elements.

### DESCRIPTION OF THE DETAILS OF THE INVENTION

A determined group of compounds named as being of the jasmonate family, adequate for this invention, compound derived from the process of metabolism, catabolism of the elements of the jasmonate family is chosen from methyl acids, jasmonic acids, 7-iso-jasmonate acid, 9,10-dihydrojasmonate acid, 2,3-dehydrojasmonate acid, 3,4-dehydrojasmonate acid, 3,7-dehydrojasmonate acid, 4,5-dehydrojasmonate acid, dehydro-7-isojasmonic acid, cucurbic acid lactones, 6-epi-cucurbic acid, 12-hydroxyjasmonic acid, 12-hydroxyjasmonic acid lactone, 11-hydrojasmonic acid, 8-dehydrojasmonic acid, homojasmonic acid, dehomojasmonic acid, 11-hydroxi-jasmonic acid, 8-hydroxi-dehydrojasmonic acid, tuberonic acid, tuberonic-O-glucopyranoside acid, 5,6-dehydrojasmonic acid, 7,8- dehydrojasmonic acid, cis-jasmonic, dehydrojasmonic, methylhydrojasmonic, jasmonic acid combined with amino acids at alkyl esters at a smaller scale.

Which has as main compound molecules by metabolism and degradation of these elements, simple or compound derived from any molecule resulting from any element of the jasmonate family obtained by processes of oxidations, reductions, hydroxydehydrogenations, hydroxylation by enzyme action such as PGDH, responsible for the breakage of these compounds similar to prostaglandin, in allylic alcohol in ketone carbonyl, formations of methylates, which can be in the simple or compound form together with other elements resulting from degradation of the molecule. These agents formed from the degradation of the jasmonate family molecule, can be isolated to be carried with the vehicles mentioned in this patent, in order to permit several specific actions of the derivate in expected target places. Instead of the entire jasmonate family molecule being used, only its active derivate is used, formed from its degradation and consequent production of new active sub products, by a simple isolated action of the formed sub molecule or by its association to other elements of metabolism and also physiologic catabolism.

The family of molecules formed by degradation also include compounds that can be prepared as post drugs by all the known processes, for example by an association with ester amide chains, and any other substance of a mineral or biological nature. The ones named as prodrugs, in this context, it regards structures that are metabolized inside the chemical environment of the body of an animal, being subject to hydrolysis reactions, such as oxidation /reduction, or catabolic or metabolic organic reactions, sometimes a blocking molecule and that can produce other compounds with metabolic or catabolic action of remaining elements, which act as medications.

Specifically, the current invention aims to obtain a useful anti cancer performance in this final product, using inclusion complexes. It can also be used in viral, bacteria, fungi, auto-immune diseases, anti aging, scaring, anti-inflammatory, analgesic, with neurologic, repairing and cosmetic activity. Members of the jasmonate family, as well as its derivates, typically very oily with low solubility (therefore very dangerous to be injected intravenously in patients, due to the risk of emboli), when they are carried, they become soluble agents presenting better pharmacokinetics, capable of administration in all types of uses, for example oral, intradermal, dermal, surgical, topic via, of the epidermis and mucus, in skin annexes, endoscopic procedures, as well as uses intraorifice, laparoscopic procedures, parenteral nutrition, in intraprocedures, lumbar puncture, cosmetic procedures, dermal sub processes, transdermal punctures, spinal or other procedures, intramuscular, inhalation, ocular, dental, as endogenous administrations, sublingual, subcutaneous, rectal use or any other mucus via use. Uses for this invention include the areas of health, food supplements, veterinary medicine, agriculture, industry and cosmetics.

The invention also includes compounds with changes in the jasmonate cyclopentanone ring, increasing or replacing, transforming it in cyclopentanones, or that add other elements to its structure, which cannot harm and can even improve its effects. The elements of the jasmonate family and its compounds can even be used for the fabrication of new compounds to be included in the nano or micro carriers.

The term micro and nano carriers applicable to all distinct transport structures, such as nano spheres, capsules in the nano form, or micro capsules can transport molecules derivate from degradation and metabolism of elements of the jasmonate family reviewed in this invention. Nano spheres are known as systems in which active principles are evenly dispersed or soluble inside a polymer matrix, the obtained system is singular, with no distinction observed between host and carried molecule .

Nano capsules are systems where it is possible to identify the two stages of compounds. In nano capsules it is possible to distinguish the difference between the two parts, the host and the guest, for example, as solid and liquid stages. In these cases substances are involved with a polymer matrix generally a nucleus isolation membrane.

Cyclodextrins (CDs) are example carriers of inclusion compounds. They are known as being cyclic oligosaccharides formed by DL (+) units of glucose bonded by a 1,4-COC chain. CDs are obtained by the enzyme degradation of amide with the CGTase gluco transferase. Native CDs are defined by the number of glucose units: α, β and γ-CDs have 6, 7 and 8 glucose units, respectively.

From the structural point of view, CDs are molecules in cone shape. In The molecular structure of CDs there are two hydrophobic and hydrophilic regions. Inside the cavity of CDs the hydrophobic character is predominant. This principle is called inclusion compound formation phenomena. In thermodynamic terms the spontaneous formation rules that lower energy systems are more probable to happen. Formation of inclusion compounds occurs because it is the lower state of energy between molecules with hydrophobic effect. Besides that, the hydrophilic part outside the CD cavity, contributes to the stability of formed inclusion compounds.

There are other cyclodextrins that are elements and molecules, natural, synthetic, semisynthetic mixtures which act as nano carriers, able to transport substances in its interior, for example, medicinal drugs. The invention includes any micro or nano particles able to encapsulate compounds derivate from the breakage of jasmonates and derivate compounds.

It is also included by it the invention of the association of jasmonate family with elements that provide specific properties, such as magnetic properties, electric, chemical properties, photo sensitivity properties, morphologic properties, acceptance properties of certain properties like biocompatibility, non rejection properties, physiologic properties, body response induction properties, protection properties, dental use properties, organic properties, organic ataxia use (lack of ability to coordinate voluntary muscular movements) the effect, all types of properties for the treatment of ataxia, radiation properties, remote guidance properties controlled from the micro or nano host, fluorescence properties, thermal properties: compounds derivate from degradation or metabolism of elements of the jasmonate family associated or modified by organic components, lipid components, metallic components, carbon components, information components, bacteria, virus, fungi, bodily fluids, lymphatic and blood elements, chemical elements used in chemotherapy, particularly metals such as zinc, copper, selenium, or mixtures of any of these alternatives.

A specific example of molecules that can form micro or nano inclusion support structures, useful for the invention are copolymer blocks such as Pluronic, a relative hydrophilic polymer and poly-ξ-caprolactone obtained from the rupture in the ξ-caprolactone ring in the presence of PEO-PPO-PEO and octane stannous catalyzer

The invention also refers to micro and nano particles including compounds derivate from the metabolism of elements of the jasmonate family interacting with other drugs such as hypoxia state inhibiting drugs in normal or cancer tissue, compounds derivate from the metabolism of elements of the jasmonate family interacting with molecules that exert its effect in the fundamental biochemical pathways - DNA synthesis, transcription, translation, genetic regulation and energy production - initially developed in conditions of anoxia, with the anaerobic glycol system which works better in hypoxia and those are the pathways which are regulated up the hypoxia cancer cell.

### EXAMPLE

The following analysis were performed with GC/MS (gas chromatography/mass spectrometry), showing possible breakages of the elements that are included in the jasmonate family and also inclusion of such elements in several nano carriers such as dendrimers and CDs for comparison of analytical behavior. Used dendrimers was PAMAM (poly amide amine). Cyclodextrin was used as β-cyclodextrin.

Preparation of inclusion complexes was performed according to Rajewski RA, Stella VJ pharmaceutical applications of cyclodextrins. 2. In delivering the in vivo drug. J. Pharm Sci 1996; 85 (11):1142-69. Inclusion compounds, or nano/micro carriers with jasmonates, can be prepared through mixing a different concentration from zero from one or more jasmonate compounds with up to a molar proportion equivalent to the host molecule. The preparation process also includes mixing the compound resulting from metabolism of the element of the jasmonate family in a water solution or other solution with acceptable pharmaceutical salts. The resulting solution is agitated until total dissolution of components in solvent. The mixing time is of some hours until the mixture reaches thermodynamic balance.

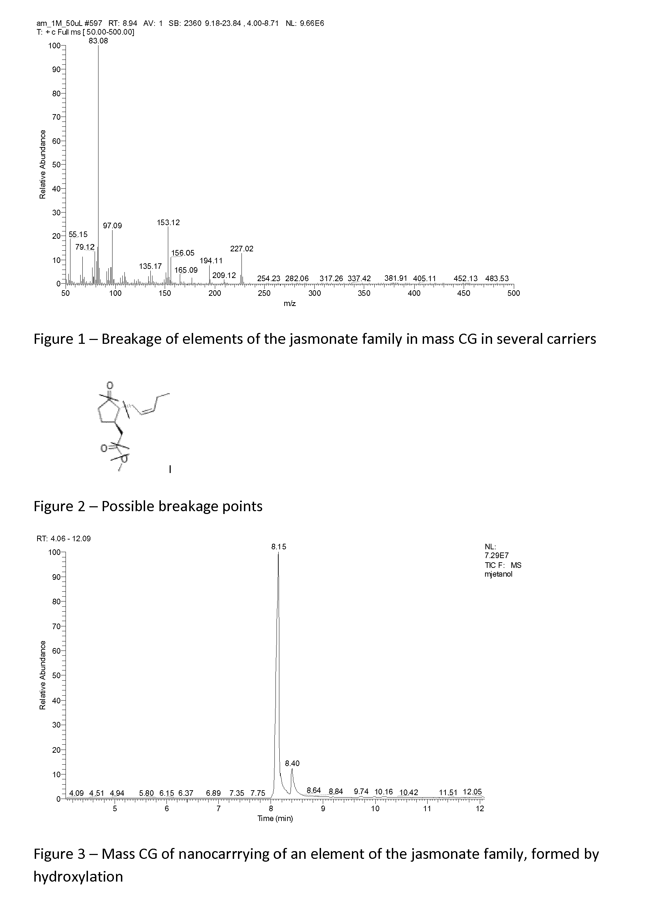

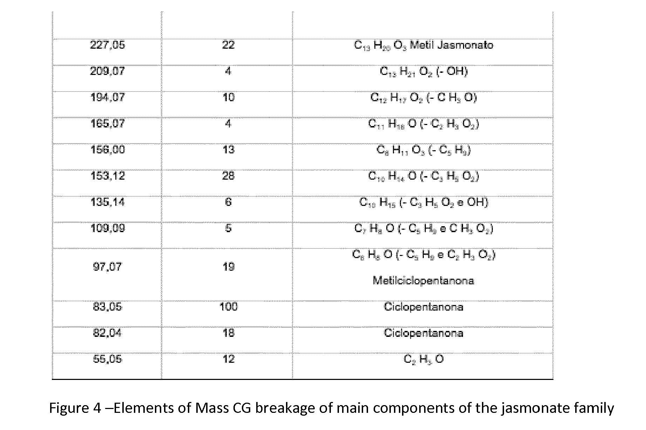

Figure 1 show the results of the analysis of nano encapsulation of one of the derivates of metabolism of methyl jasmonate, carried by dendrimers, natural cyclodextrins, gold nanospheres and Figure 2 shows the results for β-CD both with methyl jasmonate.

Experimental data was obtained with GC with the column on temperature of 50°C, injection temperature of 250°C, flow linear control way, total flow of 50,0 mL/min, flow column 1,70 mL/min, percentage regarding incorporation of methyl jasmonate within β-CD was 98-99%, and around 95% for PAMAM dendrimers.

Comparing peak 1 and peak 2, peak 2 is methyl jasmonate alone and peak 1 is the inclusion of formed compounds in both cases, peak 1 is clearly different and proves the molecular association.

## Claims

1. Pharmaceutical formulation, which has as main compound molecules by the metabolism and degradation of these elements, simple or composed: derivate from any molecule resulting from any element of the jasmonate family obtained by oxidation, reduction, hydroxide hydrogenation, hydroxylation processes, by enzyme action such as PGDH responsible for breakage of these compounds similar to prostaglandin, in allylic alcohol in ketone carbonyl, formations of methylates, which can be simple or composed together with other elements resulting from molecule degradation.

2. Pharmaceutical formulation in claim 1 where the carrier is one element or more in micrometer or nanometer size.

3. Pharmaceutical formulation in claim 1 where the composed derivates of the metabolism, catabolism process of the elements of the jasmonate family is chosen from methyl acids, jasmonic acids, 7-iso-jasmonate acid, 9,10-dihydrojasmonate acid, 2,3-dehydrojasmonate acid, 3,4-dehydrojasmonate acid, 3,7-dehydrojasmonate acid, 4,5-dehydrojasmonate acid, dehydro-7-isojasmonic acid, cucurbic acid lactones, 6-epi-cucurbic acid, 12-hydroxyjasmonic acid, 12-hydroxyjasmonic acid lactone, 11-hydrojasmonic acid, 8-dehydrojasmonic acid, homojasmonic acid, dehomojasmonic acid, 11-hydroxi-jasmonic acid, 8-hydroxi-dehydrojasmonic acid, tuberonic acid, tuberonic-O-glucopyranoside acid, 5,6-dehydrojasmonic acid, 7,8- dehydrojasmonic acid, cis-jasmonic, dehydrojasmonic, methylhydrojasmonic, jasmonic acid combined with amino acids at alkyl esters at a smaller scale.

4. Pharmaceutical formulation in claim 1 where compounds resulting from metabolism of elements of the jasmonate family are combined with any other element of physiologic metabolism.

5. Pharmaceutical formulation in claim 1 where the carrier is chosen from any of the combinations with glucose and its derivates such as fructose, lactose, as also under the inclusion form like cyclodextrins α, β and γ-cyclodextrins, dendrimers, nanospheres, nanowires, nanotubes, nanofilaments, nanoconductors, liposomes, nanorobots, biological agents, proteins, polymers and any other agent of nano o micro carriage.

6. Pharmaceutical formulation in claim 1 where the transporter are artificially modified cyclodextrins which may be pure or composed with other elements, also of different morphology and size from natural cyclodextrins.

7. Pharmaceutical formulation in claim 1 where it includes at least another chemotherapeutic agent, analgesic, anti-inflammatory, antibiotics, antifungal, antiseptic, scaring, chelator, interferon and other cellular signaling, corticosteroids, phytotherapy, bioactive elements, enzymes, as coenzymes, vegetable or animal oils, hormonal, proteins, lipids, substratum and or animal embryo elements , total venoms from several sources, and or in fractioned form, olygoelements, vitamins, amino acids, biological or plasma derivates, specific cell derivates, with modified antibodies and or monoclonal and all the scope of the cells of the immune and humoral system.

8. The use of the pharmaceutical formulation of any of the claims 1-7 in preventing and treatment of diseases and process of production of cosmetics and other therapeutic forms.

9. The use of the pharmaceutical formulation of any of the claims 1-8 in manufacturing a drug for prevention and treatment of cancer.

10. Drugs including the pharmaceutical formulation of any of the claims 1-9 and pharmaceutically accepted excipients, food, manufactures, veterinary, agricultural and industrial.
